# EUROPEAN PATENT SPECIFICATION

(11) **EP 0 874 631 B1**
(45) Date of publication and mention of the grant of the patent: **19.11.2003**
(21) Application number: 97901052.7
(22) Date of filing: 17.01.1997
(51) Int. Cl.: A61K 31/52, A61P 31/22

(54) **USE OF VALACICLOVIR FOR THE MANUFACTURE OF A MEDICAMENT FOR THE TREATMENT OF GENITAL HERPES BY A SINGLE DAILY APPLICATION**
VERWENDUNG VON VALACICLOVIR ZUR HERSTELLUNG EINES ARZNEIMITTELS ZUR BEHANDLUNG VON GENITALHERPES DURCH EINMAL TÄGLICHE VERABREICHUNG
UTILISATION DE VALACICLOVIR DANS LA FABRICATION D'UN MEDICAMENT DESTINE AU TRAITEMENT DE L'HERPES GENITAL PAR ADMINISTRATION JOURNALIERE UNIQUE

(30) Priority: 19.01.1996 US 599448
(43) Date of publication of application: 04.11.1998
(73) Proprietor: GLAXO GROUP LIMITED, Greenford, Middlesex UB6 ONN (GB)
(72) Inventor: DELEHANTY, John, Research Triangle Park, NC 27709 (US); SMILEY, Margaret, L., Research Triangle Park, NC 27709 (US)
(74) Representative: Waters, David Martin
(86) International application number: EP9700192
(87) International publication number: WO97025989

(56) References cited:
- EP-A- 0 308 065
- WO-A-95/09855
- WO-A-96/22082
- WO-A-96/22291
- ACTA DERMATOVENEROLOGICA A.P.A., vol. 4, no. 3, 1995, pages 113-116, XP000653729 C.HELLER-VITOUCH ET AL.: "Advances in the therapy of genital herpes"
- ANTIVIRAL RESEARCH, vol. 28, no. 4, 1995, pages 281-290, XP000653490 KARL R. BEUTNER: "Valacyclovir: a review of its antiviral activity, pharmacokinetic properties, and clinical efficacy"
- ANTIVIRAL CHEMISTRY & CHEMOTHERAPY, vol. 6, no. supplement, 1995, pages 39-44, XP000653501 R.J.CROOKS: "Valacyclovir - a review of its potential in the management of genital herpes"

## Description

This invention relates to the antiviral drug valaciclovir.

The compound 9-[(2-hydroxyethoxy)methyl]guanine, otherwise known as acyclovir, possesses potent antiviral activity and is widely used in the treatment and prophylaxis of viral infections in humans, particularly infections caused by the herpes group of viruses (see, for example, Schaeffer *et al,* Nature, 272, 583-585 (1978), UK Patent No. 1523865, US Patent No. 4,199,574). However, acyclovir is poorly absorbed from the gastrointestinal tract upon oral administration and this low bioavailability means that multiple high doses of oral drug may need to be administered, especially for the treatment of infections caused by less sensitive viruses in order to achieve and maintain effective anti-viral levels in the plasma.

The L-valine ester of acyclovir (2-[(2-amino-1,6-dihydro-6-oxo-9H-purin-9-yl)methoxy]ethyl L-valinate (herein referred to as valaciclovir) has been shown to produce much improved acyclovir bioavailability whilst retaining the anti-viral properties of acyclovir. A preferred form of this compound is its hydrochloride salt which is herein referred to as valaciclovir hydrochloride. Valaciclovir and its salts including the hydrochloride salt are disclosed in US Patent No. 4,957,924 (see particular example 1B), European Patent No. 0308065 (see particularly example IB) and Beauchamp et al, Antiviral Chemistry and Chemotherapy, 3(3), 157-164 (1992) (see particularly page 162 column 1). Tablets of valaciclovir are also generally disclosed in the US Patent No. 4,957,924 and European Patent No. 0308065. EP 0 308 065 A2 discloses that, for the various amino acid esters of aciclovir disclosed therein, the desired dose is preferably presented as two, three, four or more sub-doses administered at appropriate intervals throughout the day. These sub-doses may be administered in unit dosage forms for example containing 10 to 1000 mg of active ingredient per unit dosage form.

C. Heller-Vitouch et al., *Acta Dermatovenerologica A.P.A.*, 1995, 4(3), pp. 113-116 discloses the treatment of recurrent genital herpes, starting within 24 hours after appearance of clinical signs and symptoms, using valaciclovir given in a dosage of 1000 mg b.i.d. or aciclovir 200 mg given five times daily; both agents were administered throughout 5 days.

R.J.Crooks, *Antiviral Chemistry & Chemotherapy*, 1995, 6, Supplement 1, pp. 39-44 states that controlled clinical trials of valaciclovir for the acute treatment of recurrent genital herpes have demonstrated efficacy in speeding resolution of signs and symptoms, hastening lesion healing and terminating virus shedding. It states that valaciclovir increases the numbers of patients in whom lesion development is prevented, benefits achieved with twice-daily oral regimens.

S. Weller et al., *Clin. Pharmacol. Ther.*, 1993, 54, pp. 595-605 discloses the results of Phase I trials investigating the pharmacokinetics of valaciclovir after escalating single- and multiple-dose administration to normal volunteers. A single-dose study employed 8 healthy male volunteers who received valaciclovir at doses of 100, 250, 500, 750 and 1000 mg sequentially with a washout period of at least 7 days between treatments.

WO 95/09855 discloses mono- and di- valinate esters of the antiviral agents penciclovir and 9-(3-hydroxyprop-1-oxy)guanine, and discloses that doses may be administered 1 to 4 times a day or more usually 2 or 3 times a day.

Genital herpes is caused primarily by the herpes simplex type (HSV) 2 virus, but can also be caused by HSV type 1 virus. It generally recurs lifelong and therefore requires long term management. Herpes labialis is also caused by the HSV virus. The psychological burden of recurrent genital herpes means that patients prefer convenient and discreet therapeutic regimens for the long term management of the disease. In particular once daily dosage regimes ensure best patient compliance.

The current dosage regime for acyclovir in the management of genital herpes is, however, 400 mg at least twice daily, and can, in some patients, be up to five times daily.

We have now surprisingly found that from about 200 mg to above 1000 mg valaciclovir and salts thereof can now be given only once daily for the effective suppression of recurrent genital herpes.

According to a first aspect of the invention there is provided the use of valaciclovir or a pharmaceutically acceptable salt thereof in the manufacture of a medicament for the suppression of recurrent genital herpes in a human host and for administration to said human host at a once daily dose of about 200 mg to about 1000 mg of the valaciclovir or the salt thereof.

The above dosage regime may also applicable for the suppression of non-genital herpes infections, such as herpes labialis.

Preferably the daily dose is either 250 mg, 500 mg or 1000 mg, although early results indicate that 500 mg once a day is effective. This can be taken as a single or multiple unit dosage, such as a tablet.

The medicament is suitably for administration for an effective treatment period. The treatment period will depend on the patient, and lasts for as long as the patient and/or his or her physician feels that he requires valaciclovir. The treatment period can, for example, be about two months up to at least ten years. The patient would generally be reviewed annually by their physician.

Preferably also the valaciclovir is administered as valaciclovir hydrochloride, advantageously in the form of a tablet or capsule. All other aspects, characteristics and properties of valaciclovir and salts thereof discussed herein, ideally apply to the invention.

Valaciclovir and pharmaceutically acceptable salts thereof are referred to herein as the active ingredients.

The following clinical trial illustrates the invention.

OBJECTIVE: To document the number of genital herpes outbreaks during a 1-year period of suppressive therapy with valaciclovir hydrochloride in patients previously receiving chronic suppressive therapy with acyclovir.

METHODS: A total of 127 patients (81 Males, 46 Females) from 11 study sites enrolled in this open-label study. All patients had a history of recurrent genital herpes (on average 12 recurrences/year) and previously received acyclovir 400 mg twice daily as chronic suppressive therapy for up to 10 years. Upon discontinuation of acyclovir, patients received 500 mg valaciclovir hydrochloride once daily. For any genital herpes recurrence, patients self-initiated treatment with valaciclovir hydrochloride 500 mg twice daily for 5 days, then resumed once daily therapy. Patients documented recurrences in a diary and returned to the clinic every three months for drug safety and recurrence evaluation.

RESULTS: After the first three months of suppressive therapy 81% (102 of 126 patients) remained free of recurrences: following the next three months of suppressive therapy 85% (101 of 119 patients) remained free of recurrences. The total recurrence free rate after six months of suppressive therapy was 74% (93 of 126 patients). These data compare similarly to the mean quarterly and yearly recurrence free rates during the entire tenth study year of acyclovir suppression (89% and 75% respectively). Valaciclovir hydrochloride has been well tolerated for 6 months.

CONCLUSIONS: The interim results of this study indicate that valaciclovir hydrochloride 500 mg once daily may be an effective substitute for acyclovir for suppression of recurrent genital herpes. Less frequent dosing may lead to improved patient convenience and possibly to better compliance.

In another study once daily valaciclovir (as the hydrochloride salt), was evaluated as suppressive therapy in a double blind, placebo controlled multicentre clinical trial in otherwise healthy immunocompetent patients with frequently recurrent genital herpes.

A total of 382 patients with a history of >8 genital herpes recurrences per year were randomised (3:1) to treatment with once daily valaciclovir (500 mg) or placebo for 16 weeks. Patients experiencing a recurrence discontinued double blind therapy and received open label episodic valaciclovir (500 mg twice daily) for 5 days. They then continued on open label daily valaciclovir, as did those completing the 16 week double blind phase of study without evidence of a breakthrough recurrence, for a total of 48 weeks. Patients were assessed 4-weekly (double blind phase) and every 8 weeks thereafter. The primary efficacy end-point was time to first genital herpes recurrence. Preliminary results of the intent-to-treat population demonstrate a significant increase in the time to first recurrence with valaciclovir treatment compared with placebo (hazard ration 0.15 [95%CI 0.11, 0.22]; P = <0.0001, indicating an 85% reduction in recurrence rate with valaciclovir relative to placebo. Valaciclovir was well tolerated, with adverse events being infrequent and generally mild.

This controlled trial indicates that once daily valaciclovir is effective for suppression of recurrent genital herpes, thus supporting the results seen over 6 months in an open label study.

A valaciclovir composition for use in accordance with the invention may be administered by any route appropriate to the condition to be treated, but the preferred route of administration is oral. Although preferably tablets generally are included for use within the scope of the invention, for example a dispersible tablet or chewable tablet, preferably the tablet is a swallowable tablet, most preferably a film-coated swallowable tablet. It will be appreciated however, that the preferred route may vary with, for example, the condition of the recipient.

The content of drug in the oral dosage unit, e.g. a tablet, is at least about 50% w/w, preferably about 60% w/w to about 90% w/w, more preferably still about 65% w/w to about 85% w/w and most preferably about 80% w/w. Preferably the (tapped) bulk density of the drug is about 0.1 to 0.9 g/cc, more preferably 0.3 to 0.7 g/cc, more preferably still 0.34 to 0.66 g/cc, and most preferably 0.4 to 0.6g/cc. Suitably the drug is valaciclovir hydrochloride, preferably being of an anhydrous crystalline form including substantially a d-spacing pattern (derived from X-ray powder diffraction) as follows:
d spacing pattern (in Angstroms) :
   10.20 ± 0.08, 8.10 ± 0.06, 7.27 ± 0.06, 6.08 ± 0.05, 5.83 ± 0.03, 5.37 ± 0.02, 5.23 ± 0.02, 4.89 ± 0.02, 4.42 ± 0.02, 4.06 ± 0.02, 3.71 ± 0.02, 3.39 ± 0.02, 3.32 ± 0.02, 2.91 ± 0.02, 2.77 ±,0.02.

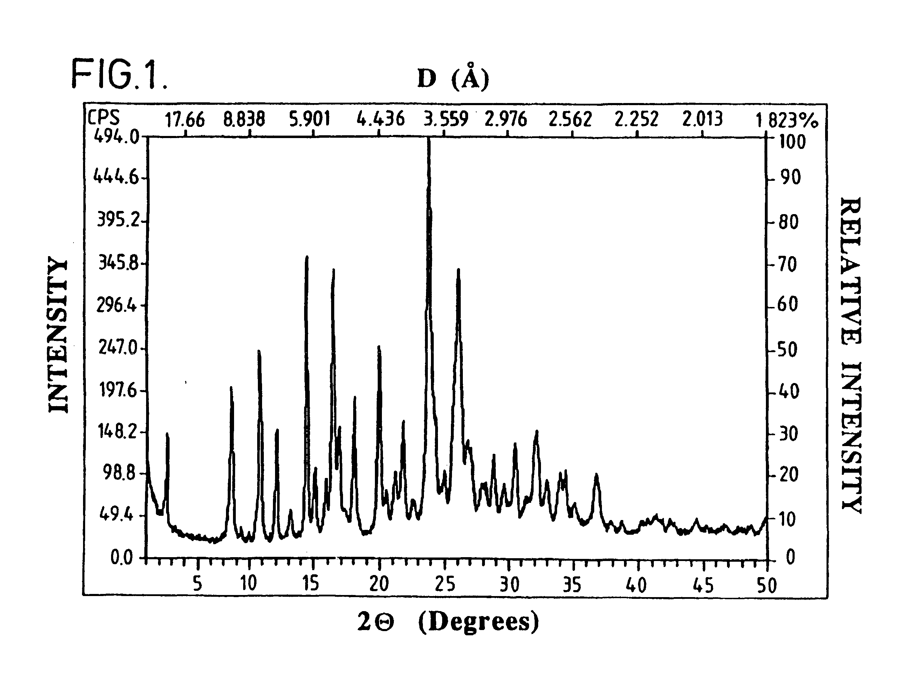

Hereinafter by "anhydrous crystalline form" according to the invention, we mean a crystalline form having substantially the same X-ray powder diffraction pattern as shown in figures 1 to 3, or having substantially the same d spacing pattern as defined above.

Preferably the crystal form purity in any such drug lot of anhydrous crystalline valaciclovir hydrochloride used for valaciclovir tablets is as least 70%, more preferably at least 80%, more preferably still at least 90% and most preferably at least 95% anhydrous crystalline valaciclovir hydrochloride (as characterised above).

In an alternative method for measuring crystal form purity, since the anhydrous crystalline form of valaciclovir hydrochloride contains substantially no water of hydration, the level of other hydrated forms of valaciclovir hydrochloride in any drug lot used for tablets can be measured by the water of hydration content. Preferably any such drug lot of anhydrous crystalline valaciclovir hydrochloride contains no more than 3% w/w, more preferably no more than 2% w/w, more preferably still not more than 1 % w/w and most preferably not more than 0. 5 % w/w water of hydration.

This water of hydration content is measured by the Karl Fischer method which is well known in the art and is described in the 1990 U.S. Pharmacopoeia at pages 1619-1621, and the European Pharmacopoeia, second edition (1992), part 2, sixteenth fascicule at v. 3.5.6-1.

A tablet of valaciclovir as used according to the invention preferably comprises at least 50% w/w valaciclovir or a salt thereof, a binding agent, a lubricant, 0.05 to 3% w/w colloidal silicon dioxide, and 3 to 30% of a cellulosic filler; wherein the valaciclovir or salt thereof is present within the granules of the tablet, the lubricant, colloidal silicon dioxide, and at least a portion of the cellulosic filler is present extragranularly.

Valaciclovir hydrochloride was made as described below:

### Example 1

### A. 2-[(2-amino-1,6-dihydro-6-oxo-9H-purin-9-yl) methoxy]ethyl-N-[(benzyloxy)carbonyl]-L-valinate

CBZ-L-valine (170 g) was dissolved in dimethylformamide (DMF) (750 ml) and cooled. A cold solution of N,N-dicyclohexyl-carbodiimide (DCC) (156.7 g) in DMF (266 ml) was added and stirred with cooling. Acyclovir (10.1 g) was added in a single portion, and then 4-(dimethylamino)pyridine (9.4 g) was added while maintaining cooling. The mixture was stirred cold overnight. A white precipitate of the by-product was then removed by filtration. The filtrate was reduced in volume by vacuum distillation and the concentrate treated with water (663 ml) then heated to 70°C. The suspension was cooled to 20°C, filtered and the solid washed with water.

The damp, crude material was then purified by recrystallisation from denatured alcohol (1.2 litres) to afford the title compound as a damp white crystalline solid (281.5 g).

### B. 2-[(2-amino-1,6-dihydro-6-oxo-9H-purin-9-yl) methoxy]ethyl-L-valinate hydrochloride

2-[(2-amino-1,6-dihydro-6-oxo-9H-purin-9-yl)methoxy]ethyl-N-[(benzyloxy)carbonyl]-L-valinate (175 g) was charged to aqueous denatured alcohol (335 ml/795 ml) and heated to reflux. The solution was then cooled to 40°C. The suspension was treated with 5% palladium on carbon catalyst (35 g wet weight 50% wet with water) then formic acid (30.6 ml of 90% w/w) added over 1 hour. The reaction mixture was stirred for a further 1 hour then a second charge of formic acid made (19.5 ml) and the mixture filtered to remove the catalyst. The filter cake was washed with denatured alcohol and the combined filtrates were treated with concentrated hydrochloric acid (33.7 ml) and the resultant mixture was concentrated by vacuum distillation.

Acetone (1295 ml) was then added over 15 minutes and the suspension stirred for 1 hour before filtering off the product. The solid was then slurried with acetone (circa. 530 ml) , refiltered and dried at 60°C *in vacuo* to give the title compound (1123 g : 81.6%).

A 15 g sample of this material was combined with denatured alcohol (circa. 7 ml), to moisten and was heated with agitation at 60°C overnight in a closed flask to avoid loss of alcohol and maintain the dampness of the mixture. The mixture was then dried at 60°C in *vacuo* to afford the product as the desired morphic form.

### Physical Data:

### Karl Fischer value : 0.9% w/w water.

The X-ray powder diffraction patterns of the product of example 1B are shown in Figure 1 of the accompanying drawings.

The d spacings and further X-ray diffraction data are shown in Table 1.

**Table 1**

| Peak No: | Angle (degrees) | Peak (counts) | d Spacing pattern (Å) | Error in d (± Å) | I/Imax (%) |
|---|---|---|---|---|---|
| 1 | 3.56 | 680 | 24.8 | 0.5 | 24 |
| 2 | 8.62 | 1151 | 10.25 | 0.08 | 39 |
| 3 | 9.42 | 87 | 9.38 | 0.07 | 3 |
| 4 | 10.86 | 1438 | 8.14 | 0.06 | 49 |
| 5 | 12.10 | 835 | 7.31 | 0.06 | 28 |
| 6 | 13.22 | 198 | 6.69 | 0.05 | 6 |
| 7 | 14.49 | 2172 | 6.11 | 0.05 | 75 |
| 8 | 15.12 | 455 | 5.85 | 0.03 | 15 |
| 9 | 15.90 | 352 | 5.57 | 0.02 | 12 |
| 10 | 16.45 | 1969 | 5.38 | 0.02 | 68 |
| 11 | 16.90 | 744 | 5.24 | 0.02 | 25 |
| 12 | 17.33 | 119 | 5.11 | 0.02 | 4 |
| 13 | 18.12 | 1013 | 4.89 | 0.02 | 35 |
| 14 | 22.71 | 1429 | 4.43 | 0.02 | 49 |
| 15 | 20.55 | 256 | 4.32 | 0.02 | 8 |
| 16 | 21.21 | 370 | 4.19 | 0.02 | 12 |
| 17 | 21.83 | 753 | 4.07 | 0.02 | 26 |
| 18 | 22.71 | 95 | 3.91 | 0.02 | 3 |
| 19 | 23.95 | 2893 | 3.71 | 0.02 | 100 |
| 20 | 25.10 | 171 | 3.54 | 0.02 | 5 |
| 21 | 26.21 | 1784 | 3.40 | 0.02 | 61 |
| 22 | 26.89 | 428 | 3.31 | 0.02 | 14 |
| 23 | 27.08 | 373 | 3.29 | 0.02 | 12 |
| 24 | 28.02 | 158 | 3.18 | 0.02 | 5 |
| 25 | 28.27 | 161 | 3.15 | 0.02 | 5 |
| 26 | 28.91 | 391 | 3.09 | 0.02 | 13 |
| 27 | 29.68 | 191 | 3.01 | 0.02 | 6 |
| 28 | 30.55 | 502 | 2.92 | 0.02 | 17 |
| 29 | 31.34 | 110 | 2.85 | 0.02 | 3 |
| 30 | 31.58 | 98 | 2.83 | 0.02 | 3 |
| 31 | 32.13 | 597 | 2.78 | 0.02 | 20 |
| 32 | 32.96 | 260 | 2.72 | 0.02 | 8 |
| 33 | 33.99 | 344 | 2.64 | 0.02 | 11 |
| 34 | 34.38 | 374 | 2.61 | 0.02 | 12 |
| 35 | 35.12 | 141 | 2.55 | 0.02 | 4 |
| 36 | 36.78 | 408 | 2.44 | 0.02 | 14 |
| 37 | 38.71 | 101 | 2.32 | 0.02 | 3 |
| I/Imax = (peak height/max. peak ht) x 100 | | | | | |

The powder sample used to produce the above X-ray diffraction data was prepared by an equivalent method as the powder sample used to produce the X-ray diffraction date of table 2 (described hereinafter) except that for the above data the following preparation was used to prepare the powder sample.

The sample was prepared by milling 1 g of sample in a plastic cup using two acrylic balls for 5 minutes with a Chemplex Spectromill. The samples were then back packed against a glass slide to a depth of 2 mm.

The X-ray diffraction scan was obtained using a Scintag PADV diffractometer in the step scan mode at 0.02° per step and a 10 second count per step. The sample holder was spun at 1 rotation per second during the scan. Additional setting as described below.

| | |
|---|---|
| X-ray generator | 45 kV, 40 mA |
| Radiation | Copper K alpha radiation |
| Fixed divergent slit | 1 mm |
| Incident scatter slit | 2 mm |
| Diffracted scatter slit | 0.5 mm |
| Receiving slit | 0.3 mm |
| Goniometer radius | 235 mm |
| Detector | Scintillation with a graphite monochromator. |

The peak intensities are reported as absolute counts of the peak top. The intensity units on the X-ray diffraction plot are counts/sec. The absolute counts = counts/sec x count time = counts/sec x 10 sec. The peak intensities in the table have been corrected for background and copper K alpha II X-ray wavelength contribution.

### Example 2

### A. 2-[(2-amino-1,6-dihydro-6-oxo-9H-purin-9-yl) methoxy]ethyl-N-[(benzyloxy)carbonyl]-L-valinate

CBZ-L-valine (167 g) was dissolved in dimethylformamide (DMF) (750 ml) and cooled to 0.5°C. A cold solution of N,N-dicyclohexylcarbodiimide (DCC) (153.5 g) in DMF (266 ml) was added followed by acyclovir (111.7 g) in a single portion. 4 (Dimethylamino)pyridine (9.4 g) was then added and the mixture stirred cold overnight. A white precipitate of the by-product was then removed by filtration. The solvent was partially removed by vacuum distillation and the concentrate treated with water (663 ml) then heated to 70°C. The suspension was cooled to 20°C, filtered and the solid washed with water.

The damp, crude material was then purified by recrystallisation from denatured alcohol (1.2 litres) to afford the title compound as a damp white crystalline solid (215.3 g).

### B. 2-[(2-amino-1,6-dihydro-6-oxo-9H-purin-9-yl) methoxy]ethyl-L-valinate hydrochloride

2-[(2-amino-1,6-dihydro-6-oxo-9H-purin-9-yl)methoxy]ethyl-N-[(benzyloxy)carbonyl]-L-valinate (200 g) was charged to aqueous denatured alcohol (382 ml / 908 ml) and heated to reflux to dissolve solids. The solution was cooled to 40°C. The suspension was treated with a 50% w/w paste of 5% palladium on carbon catalyst and water (40 g) then formic acid (96% w/w : 32.8 ml) added over 1 hour. The reaction mixture was stirred for a further 1 hour then a second charge of formic acid made (20.88 ml) and the mixture filtered to remove the catalyst. The filtrate was treated with concentrated hydrochloric acid (38.56 ml) and the resultant mixture was concentrated under vacuum.

Acetone (1480 ml) was then added over 15 minutes and the suspension stirred for 1 hour before filtering off the product. The solid was then slurried with acetone (ca. 500 ml), refiltered and dried at 60°C in *vacuo* to give the title compound (137.75 g : 87.6%).

A 10 g sample of this material was combined with denatured alcohol (3.5 ml), heated at 60°C for several hours and the solvent then removed in *vacuo* to afford the product as the desired morphic form.

Crystal Form Purity: the sample of example 2(B) contained above 90% of the anhydrous crystalline form valaciclovir.

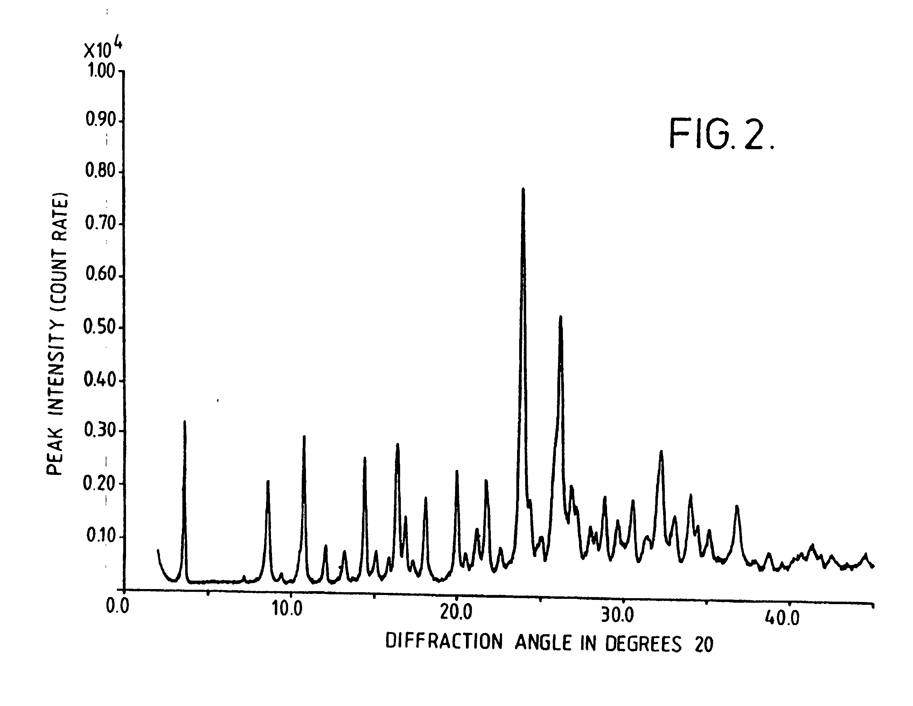

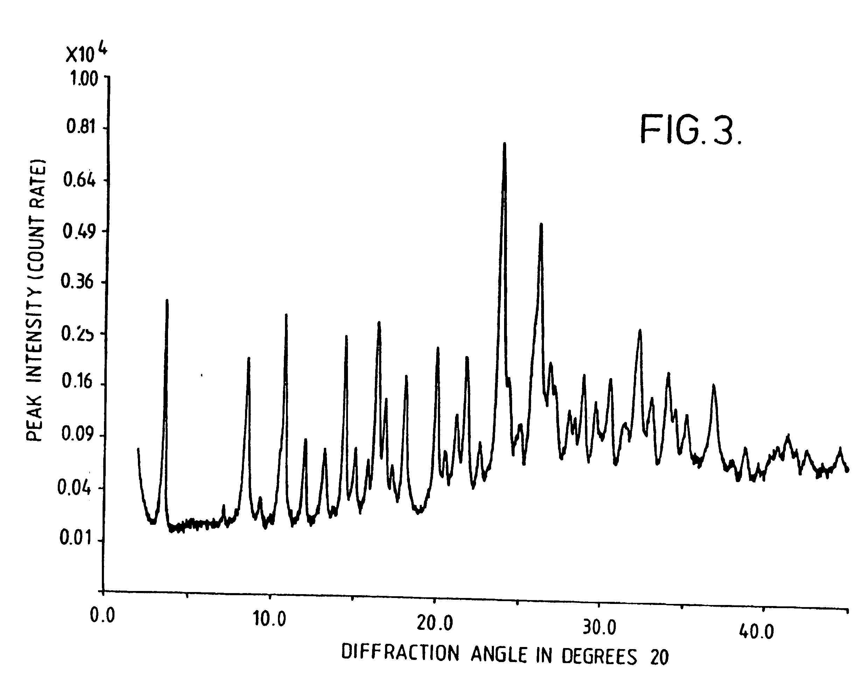

The X-ray powder diffraction patterns of the product of example 2(B) are shown in Figures 2 and 3 of the accompanying drawings in which:-
Fig 2 is a linear plot X-ray diffractogram; and
Fig 3 is a square root plot X-ray diffractogram.

The d spacings and further X-ray diffraction data are shown in Table 2

**Table 2**

| Peak No: | Angle (degrees) | Peak (counts) | d Spacing pattern (Å) | I/Imax (%) |
|---|---|---|---|---|
| 1 | 3.62 | 2673 | 24.40 | 35 |
| 2 | 7.21 | 119 | 12.26 | 2 |
| 3 | 8.64 | 1910 | 10.22 | 25 |
| 4 | 9.43 | 180 | 9.37 | 2 |
| 5 | 10.86 | 2652 | 8.14 | 35 |
| 6 | 12.12 | 734 | 7.30 | 10 |
| 7 | 13.24 | 615 | 6.68 | 8 |
| 8 | 13.77 | 106 | 6.42 | 1 |
| 9 | 14.50 | 2333 | 6.11 | 31 |
| 10 | 15.14 | 635 | 5.85 | 8 |
| 11 | 15.89 | 511 | 5.57 | 7 |
| 12 | 16.44 | 2652 | 5.39 | 35 |
| 13 | 16.90 | 1267 | 5.24 | 17 |
| 14 | 17.33 | 475 | 5.11 | 6 |
| 15 | 18.13 | 1648 | 4.89 | 22 |
| 16 | 20.05 | 2172 | 4.43 | 28 |
| 17 | 20.56 | 640 | 4.32 | 8 |
| 18 | 21.20 | 1096 | 4.19 | 14 |
| 19 | 21.78 | 2034 | 4.08 | 27 |
| 20 | 21.90 | 1384 | 4.06 | 18 |
| 21 | 22.66 | 729 | 3.92 | 10 |
| 22 | 23.94 | 7621 | 3.71 | 100 |
| 23 | 24.39 | 1624 | 3.65 | 21 |
| 24 | 25.11 | 967 | 3.54 | 13 |
| 25 | 25.86 | 2460 | 3.44 | 32 |
| 26 | 26.21 | 5127 | 3.40 | 67 |
| 27 | 26.82 | 1892 | 3.32 | 25 |
| 28 | 26.89 | 1927 | 3.31 | 25 |
| 29 | 27.19 | 1429 | 3.28 | 19 |
| 30 | 27.99 | 1156 | 3.18 | 15 |
| 31 | 28.35 | 1076 | 3.15 | 14 |
| 32 | 28.87 | 1722 | 3.09 | 23 |
| 33 | 28.94 | 1529 | 3.08 | 20 |
| 34 | 29.62 | 1274 | 3.01 | 17 |
| 35 | 30.56 | 1673 | 2.92 | 22 |
| 36 | 31.30 | 999 | 2.86 | 13 |
| 37 | 32.25 | 2570 | 2.77 | 34 |
| 38 | 33.04 | 1376 | 2.71 | 18 |
| 39 | 34.00 | 1806 | 2.63 | 24 |
| 40 | 34.45 | 1225 | 2.60 | 16 |
| 41 | 35.13 | 1149 | 2.55 | 15 |
| 42 | 36.77 | 1600 | 2.44 | 21 |
| 43 | 38.01 | 576 | 2.37 | 8 |
| 44 | 38.76 | 729 | 2.32 | 10 |
| 45 | 39.52 | 524 | 2.28 | 7 |
| 46 | 40.70 | 751 | 2.22 | 10 |
| 47 | 41.28 | 870 | 2.19 | 11 |
| 48 | 41.88 | 686 | 2.16 | 9 |
| 49 | 42.47 | 718 | 2.13 | 9 |
| 50 | 43.40 | 548 | 2.08 | 7 |
| 51 | 44.53 | 729 | 2.03 | 10 |

The diffraction patterns of the product of example 2B were generated on a Phillips PW1800 Automatic X-ray Powder Diffractometer using a scan of 2 to 45 2θ with step intervals of 0.02 degrees and an integration time of 4 seconds per step.

Generator settings: 40 KV, 45 mA, Cu alpha 1,2 wavelengths: 1.54060, 1.54439 Å; Step size, sample time: 0.020 deg, 4.00 s, 0,005 deg/s; monochromator used: yes; divergence slit: automatic (irradiated sample length: 10.0 mm); peak angle range: 2.000 - 45.000 deg; range in D spacing: 44.1372 - 2.01289 Å; peak position criterion: top of smoothed data; cryst peak width range: 0.00 - 2.00 deg; minimum peak significance: 0.75 maximum intensity: 7621 cts, 1905.3 cps.

The powder sample was prepared as follows:

A 1 gram portion of valaciclovir hydrochloride was transferred to a Retsch 10 ml polystyrol container ref 31-762 containing 2 acrylic balls ref 26-253 and was then ground to a very fine powder using a Retsch MM2 miser mill set at 100% power for five minutes. The ground powder was back loaded into a Philips PW1811/10 sample holder which had been placed inverted on a perfectly smooth surface (e.g. that afforded by a glass plate or a highly polished metal sheet). The powder was then packed into the holder and further powder added and packed until the holder was full. A Philips PW 1811 00 bottom plate was then clamped into the holder and the entire assembly was then inverted before removing the glass/metal plate in an upwards direction to reveal the smooth sample surface which was flush with that of the holder.

Formulations of valaciclovir which can be used in accordance with the invention are shown in the following examples.

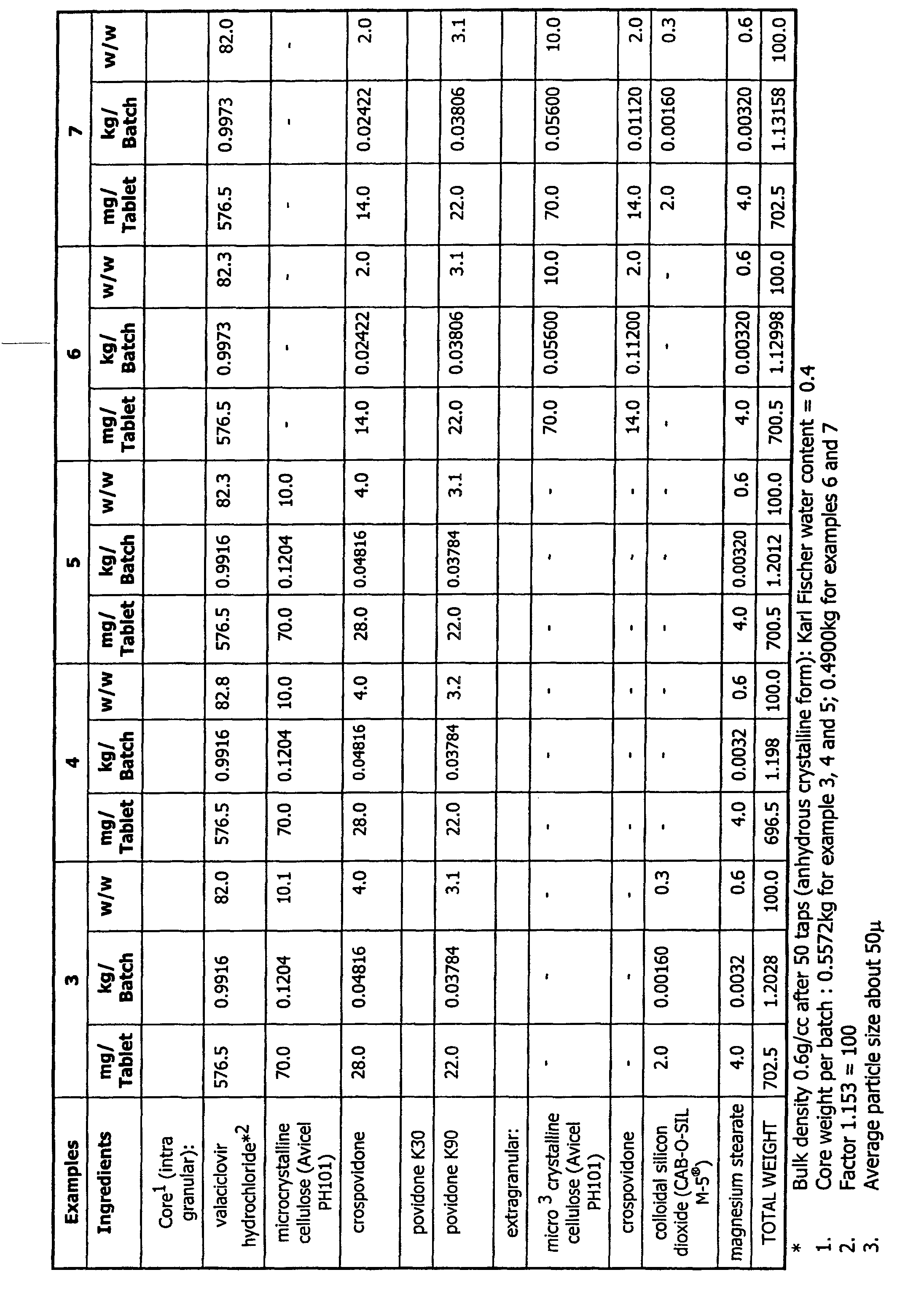

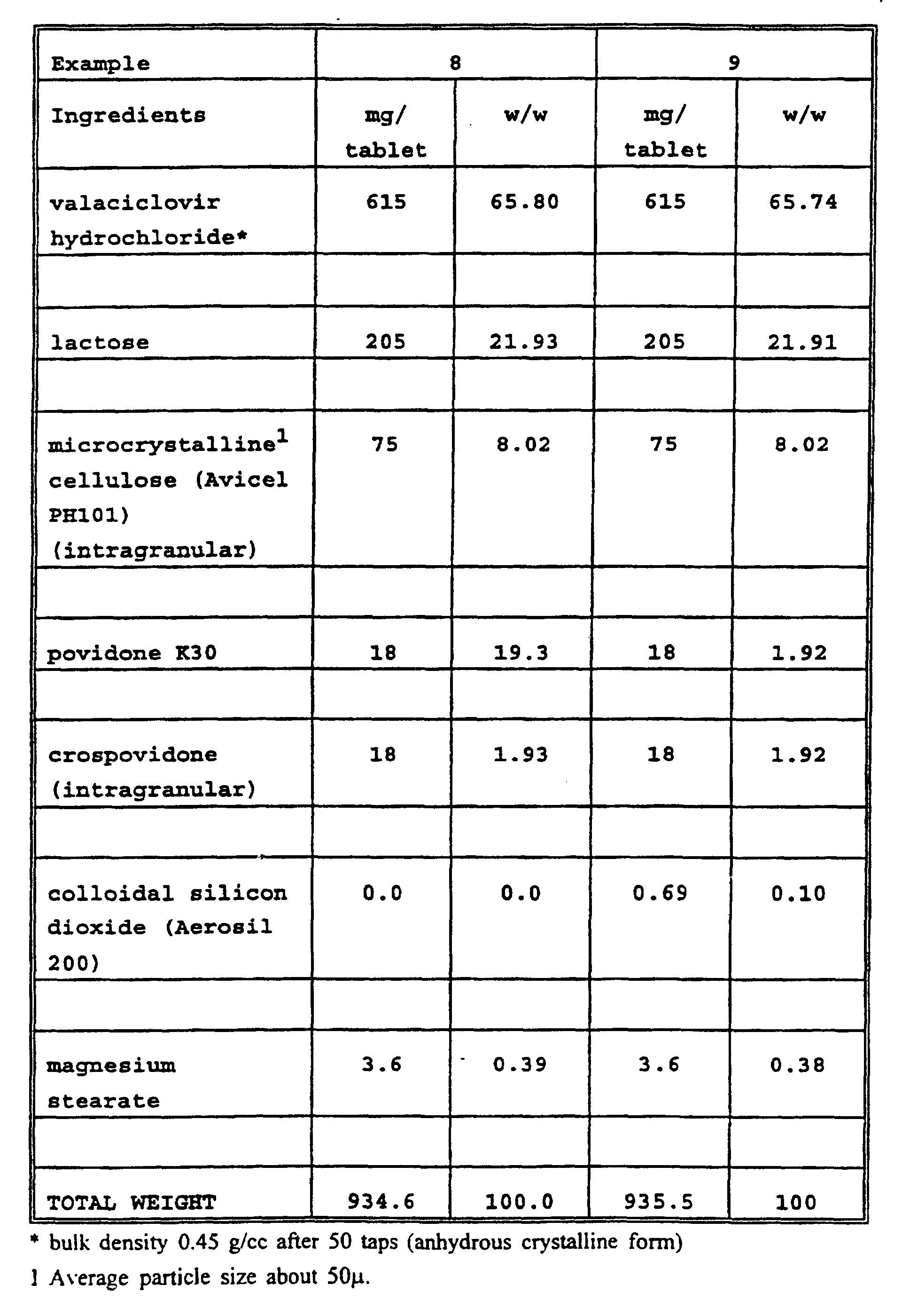

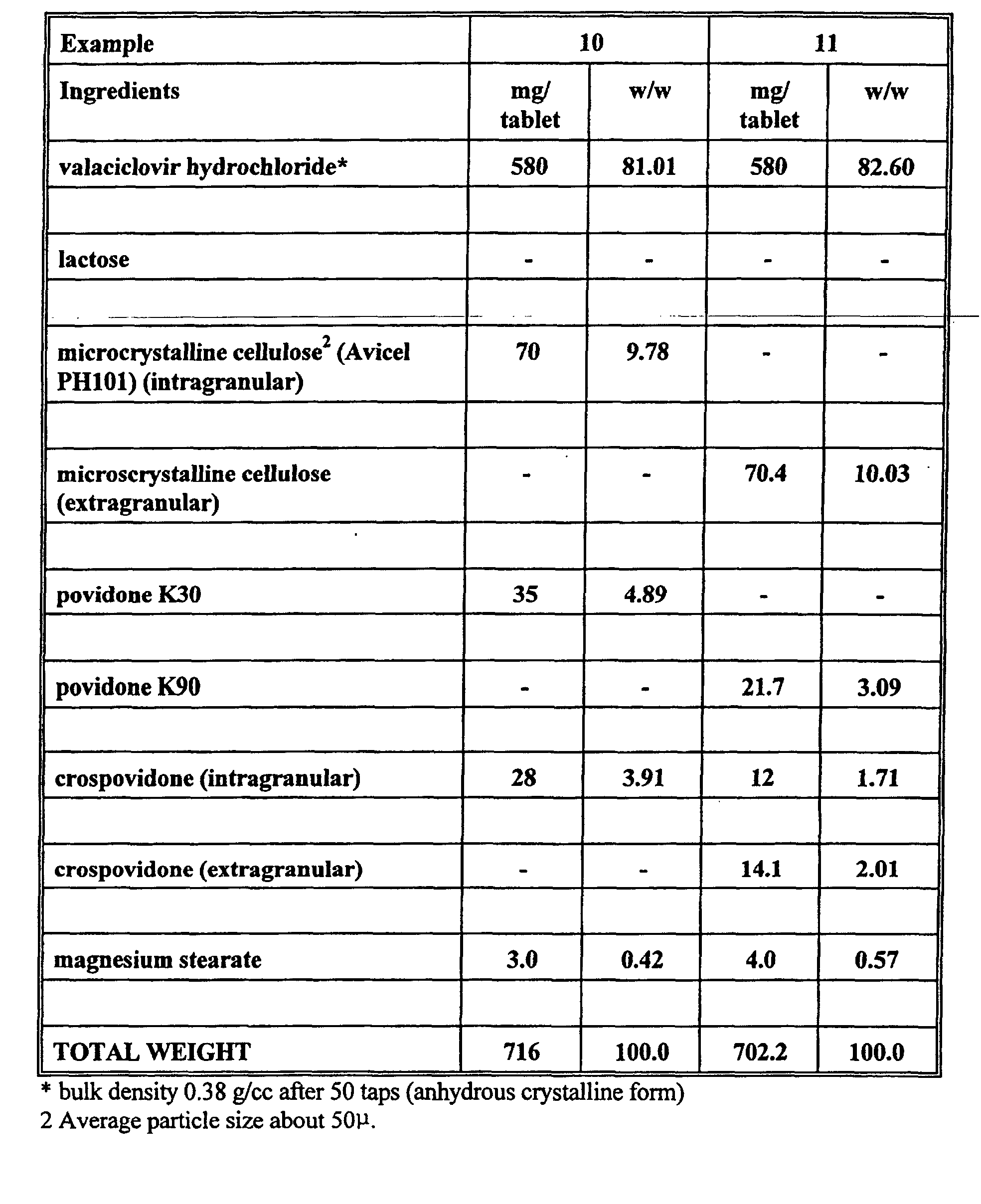

The tablets of the examples were made as disclosed below.

### Examples 3 to 7

- Step 1.: The core ingredients were sifted with a 20 mesh hand screen, and then blended in an appropriately sized V-shell blender for 10 minutes.
- Step 2.: The blended powders from Step 1 were then granulated in a 10 litre high shear mixer (model-SP1) by adding pure water while mixing. Approximately 11-14% water, w/w of the core ingredients was then added and the mixture massed for 3 to 4½ minutes.
- Step 3.: The granule from Step 2 was dried in a tray (examples 5, 6 and 7) or vacuum (examples 3 and 4) drier (model-SP1) at a temperature of 50°C to an acceptable moisture content of approximately 1.0 to 2.0 % L.O.D.
- Step 4.: The remaining ingredients were sifted through a 20 mesh screen and added to the core ingredients of step 3, and then the mixture was sifted using a Comil Model 197 AS fitted with a 0.062" screen.
- Step 5.: The mixture was then blended in an appropriately sized V-shell blender for 5 minutes.
- Step 6: The blended granule from Step 5 was compressed on a Manesty Beta Press fitted with capsule shaped tooling, 18.25 mm x 7.14 mm, at a compression weight of approximately 700 mg and a compression force of about 14.5 to 18 kN .
- Step 7: The tablets can then optionally be film coated by using standard methods such as using white colour concentrate, methylhydroxypropykellulose, titanium dioxide, polyethylene glycol and polysorbate.

Hardness (crushing force through the long axis) was measured using a Key hardness tester, Model HT-300. Friability (percent weight loss after 100, six inch drops) was measured in accordance with the USP no. 23, 1995, p1981 at monograph 1216, using an Erweka friability tester, Model TA-3. Physical properties were measured at comparable compression forces. The disintegration time was measured in accordance with the monograph in USP 23 (1995) at page 1790.

### Examples 8 and 9

Step 1. The following ingredients as shown were sifted with a hand screen.

| 30 Mesh | |
|---|---|
| valaciclovir hydrochloride | 5.289 kg |
| lactose | 1.763 kg |
| microcrystalline Cellulose | 0.6450 kg |
| povidone K30 | 0. 1548 kg |
| crospovidone | 0. 1548 kg |

| 60 Mesh | |
|---|---|
| magnesium stearate | 0.03096 kg |
| colloidal silicon dioxide (CSD) | 0.002598 kg |

Step 2. The 30 mesh sifted ingredients from Step I were then blended, excluding the povidone, in a 1 cubic foot V-shell blender for 10 minutes.
Step 3. 1.540 kg of SD3A alcohol (ethanol denatured with 5% methanol) was then mixed with 0.6600 kg of purified water and the screened povidone, 0.1548 kg, was dissolved in 0.6192 kg of the mixed solvents by hand stirring.
Step 4. The blended powders from Step 2 were then granulated in a 1 cubic foot Littleford Lodige mixer by adding the dissolved povidone while mixing. 1.315 kg of more mixed solvent was added and the mixture massed for seven minutes total as shown below.

| | |
|---|---|
| Ploughs 7 min | Choppers 6.5 min |

Step 5. The granule from Step 4 was then dried in a Fluid Bed Dryer (Glatt GPCG5) with an inlet air temperature of 50°C to any acceptable moisture content of approximately 1.0 to 3.0% L.O.D.
Step 6. The granule from Step 5 was then sifted using a Fitz Mill Model M fitted with a 30 mesh screen, with knives forward, operating at medium speed.
Step 7. The screened magnesium stearate from step 1 was added to the granule from Step 6 and blended for 5 minutes using the blender from Step 2. This was labelled as example 10 (2.650kg).
Step 8. Part of the blended granule from Step 7 was compressed on a Manesty Beta Press fitted with oval tooling, 19.1 mm x 10.2 mm, at a compression weight of approximately 934.6 mg.
Step 9. The remainder of the lubricated granule 2.650 kg (from Step 7) was weighed and the sifted CSD from step I added, then dispersed by hand and the mixture blended for 5 minutes in the blender from Step 3. This portion was labelled as Example 11. The mixture was compressed to form tablets.

Examples 10 and 11 were manufactured in a substantially similar manner to Examples 8 and 9 with the following exceptions.
1. All ingredient were sifted through a 20 mesh sieve.
2. Drug and intragranular ingredients were blended for 10 minutes.
3. The amounts of water and SD3A alcohol were adjusted for the difference in batch size.
4. Dried granule was milled using a Comil Model 197AS with 0.062" screen.
5. Example 11 was dried in a tray drier.
6. The magnesium stearate was blended for 10 minutes after 10 minutes preblend of the milled granule and other ingredients.

### Example 12 Clinical Trial Results

Objective: To determine the efficacy and safety of 4 doses of oral valaciclovir compared with aciclovir and placebo for the suppression of genital herpes in otherwise healthy patients.

Methods: This study was a multi-centre, international, six-arm, parallel group, randomised, double-blind comparison of the efficacy and safety of 4 doses of oral valaciclovir with oral aciclovir and placebo for the suppression of recurrent episodes of genital herpes simplex virus (HSV) infection in immunocompetent patients. A total of 1279 patients with a history of recurrent genital herpes were randomised into one of 6 treatment arms with a 2:2:2:2:2:1 treatment allocation and followed for 52 weeks. Patients visited the clinic at monthly intervals and on Days 1 and 5 of a genital HSV recurrence. Safety was assessed by adverse experience reporting at each visit and by clinical chemistry and haematology testing every 3 or 6 months respectively.

274 patients received 250 mg twice daily valaciclovir (reference example); 269 patients received 1000 mg once daily valaciclovir; 266 patients received 500 mg once daily valaciclovir; 269 patients received 250 mg once daily valaciclovir; 267 patients received 400 mg twice daily aciclovir; and 134 patients received placebo.

Results: Aciclovir, valaciclovir 1000 mg once daily and 250 mg twice daily prevented or delayed 78-79% of recurrences experienced by placebo patients during the study period. Valaciclovir 500 mg once daily prevented or delayed 71% of recurrences but this fell to 54% for patients who received valaciclovir 250 mg once daily. A dose-response relationship was seen across the once-daily treatment arms confirming that increasing the valaciclovir once daily dose provided an increase in efficacy. With regard to the patients who were recurrence-free after 12 months, approximately half the patients in the aciclovir, valaciclovir 1000 mg once daily and valaciclovir 250 mg twice daily groups had not had a recurrence during the year compared with only 5% of the patients in the placebo group. Forty percent of the valaciclovir 500 mg once daily group were still recurrence-free at 12 months but only 22% of patients in the valaciclovir 250 mg once daily group remained recurrence-free during the study period. The safety profiles of valaciclovir, aciclovir and placebo were similar. Adverse experiences were generally infrequent and mild in intensity.

Conclusions: Valaciclovir 250 mg, 500 mg and 1000 mg once daily and 250 mg twice daily can significantly prevent or delay the recurrence of genital HSV over a period of 1 year. All doses of valaciclovir provide the same degree of safety seen with aciclovir.

## Claims

1. Use of valaciclovir or a pharmaceutically acceptable salt thereof in the manufacture of a medicament for the suppression of recurrent genital herpes in a human host and for administration to said human host at a once daily dose of 200 mg to 1000 mg of the valaciclovir or the salt thereof.

2. Use as claimed in Claim 1 wherein the medicament is for oral administration.

3. Use as claimed in claim 2 wherein the medicament is a tablet.

4. Use as claimed in claim 1, 2 or 3 wherein the once daily dose is 250 mg, 500 mg or 1000 mg.

5. Use as claimed in claim 1, 2 or 3 wherein the once daily dose is 500 mg.

6. Use as claimed in claim 1, 2 or 3 wherein the once daily dose is 1000 mg.

7. Use as claimed in any one of Claims 1 to 6 wherein the valaciclovir or the pharmaceutical acceptable salt thereof is valaciclovir hydrochloride.

8. Use as claimed in any one of Claims 1 to 7 wherein the medicament is for administration for a treatment period of from about two months up to at least ten years.

## Patentansprüche

1. Verwendung von Valaciclovir oder eines pharmazeutisch verträglichen Salzes davon zur Herstellung eines Medikaments zur Suppression von wiederholt auftretendem Genitalherpes in einem menschlichen Wirt und zur Verabreichung an den menschlichen Wirt in einer einmaligen Tagesdosis von 200 mg bis 1000 mg des Valaciclovirs oder des Salzes davon.

2. Verwendung gemäß Anspruch 1, wobei das Medikament zur oralen Verabreichung ist.

3. Verwendung gemäß Anspruch 2, wobei das Medikament eine Tablette ist.

4. Verwendung gemäß Anspruch 1, 2 oder 3, wobei die einmalige Tagesdosis 250 mg, 500 mg oder 1000 mg beträgt.

5. Verwendung gemäß Anspruch 1, 2 oder 3, wobei die einmalige Tagesdosis 500 mg beträgt.

6. Verwendung gemäß Anspruch 1, 2 oder 3, wobei die einmalige Tagesdosis 1000 mg beträgt.

7. Verwendung gemäß einem der Ansprüche 1 bis 6, wobei das Valaciclovir oder das pharmazeutisch verträgliche Salz davon Valaciclovirhydrochlorid ist.

8. Verwendung gemäß einem der Ansprüche 1 bis 7, wobei das Medikament zur Verabreichung für einen Behandlungszeitraum von etwa 2 Monaten bis zu mindestens 10 Jahren ist.

## Revendications

1. Utilisation de valaciclovir ou d'un de ses sels pharmaceutiquement acceptables dans la production d'un médicament destiné à la suppression de l'herpès génital récidivant chez un hôte humain et à l'administration audit hôte humain à une dose quotidienne en une fois de 200 mg à 1000 mg de valaciclovir ou de son sel.

2. Utilisation suivant la revendication 1, dans laquelle le médicament est destiné à l'administration orale.

3. Utilisation suivant la revendication 2, dans laquelle le médicament est un comprimé.

4. Utilisation suivant la revendication 1, 2 ou 3, dans laquelle la dose quotidienne en une fois est égale à 250 mg, 500 mg ou 1000 mg.

5. Utilisation suivant la revendication 1, 2 ou 3, dans laquelle la dose quotidienne en une fois est égale à 500 mg.

6. Utilisation suivant la revendication 1, 2 ou 3, dans laquelle la dose quotidienne en une fois est égale à 1000 mg.

7. Utilisation suivant l'une quelconque des revendications 1 à 6, dans laquelle le valaciclovir ou son sel pharmaceutiquement acceptable est le chlorhydrate de valaciclovir.

8. Utilisation suivant l'une quelconque des revendications 1 à 7, dans laquelle le médicament est destiné à l'administration pendant une période de traitement d'environ 2 mois jusqu'à au moins 10 ans.
